(19) Europäisches Patentamt
European Patent Office
Office européen des brevets

(11) **EP 4 338 674 B1**

(12) **EUROPÄISCHE PATENTSCHRIFT**

(45) Veröffentlichungstag und Bekanntmachung des Hinweises auf die Patenterteilung:
**11.02.2026   Patentblatt 2026/07**

(21) Anmeldenummer: **22195622.0**

(22) Anmeldetag: **14.09.2022**

(51) Internationale Patentklassifikation (IPC):
*A61B 6/00* (2024.01)       *G06T 7/00* (2017.01)

(52) Gemeinsame Patentklassifikation (CPC):
**A61B 6/502; A61B 6/481; A61B 6/482; A61B 6/5217; G06T 7/0012; G06T 7/44;** G06T 2207/10116; G06T 2207/20036; G06T 2207/30068

(54) **VERFAHREN UND SYSTEM ZUR BESTIMMUNG DES BPE IN EINER KONTRASTMITTELVERSTÄRKTEN RÖNTGENUNTERSUCHUNG EINER BRUST**

METHOD AND SYSTEM FOR DETERMINING BPE IN A CONTRAST AGENT-ENHANCED BREAST X-RAY EXAMINATION

PROCÉDÉ ET SYSTÈME POUR DÉTERMINER LA BPE DANS UN EXAMEN RADIOGRAPHIQUE D'UN SEIN AMÉLIORÉ PAR UN AGENT DE CONTRASTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.03.2024   Patentblatt 2024/12**

(73) Patentinhaber: **Siemens Healthineers AG**
**91301 Forchheim (DE)**

(72) Erfinder:
• **Fieselmann, Andreas**
  **91052 Erlangen (DE)**
• **Hörnig, Mathias**
  **91096 Möhrendorf (DE)**

(74) Vertreter: **Siemens Healthineers**
**Patent Attorneys**
**Postfach 22 16 34**
**80506 München (DE)**

(56) Entgegenhaltungen:
**WO-A1-2022/003656     US-A1- 2021 097 677**

**Beschreibung**

Die Erfindung betrifft ein Verfahren und System zur Bestimmung des BPE in einer kontrastmittelverstärkten Röntgenuntersuchung einer Brust sowie eine dafür geeignete Steuereinrichtung und ein entsprechendes Mammographiesystem. Die Erfindung befasst sich insbesondere mit einer Automatisierten Beurteilung von BPE bei einer kontrastmittelverstärkten Zwei-Energie Röntgenuntersuchung der Brust.

Das Hintergrundenhancement (im deutschen auch oft englisch als "Background Parenchymal Enhancement" bezeichnet, weshalb hier die typischerweise verwendete Abkürzung "BPE" verwendet wird) ist ein häufiges Bildmerkmal in der kontrastmittelverstärkten Magnetresonanztomographie (ce-MRT) und der kontrastmittelverstärkten 2D-Dual-Energie-Mammographie (auch bekannt als CEDEM für "Contrast-Enhanced Dual Energy Mammography).

BPE gilt als Risikofaktor für Brustkrebs. Savaridas et al. ("Could parenchymal enhancement on contrast-enhanced spectral mammography (CESM) represent a new breast cancer risk factor? Correlation with known radiology risk factors", Clinical Radiology, vol. 72(12), 2017) merken dazu an, dass die Einstufung des BPE-Grads auf CESM eine nützliche Ergänzung zu den Instrumenten zur Risikobewertung von Brustkrebs sein könnten. Sorin et al. ("Background Parenchymal Enhancement at Contrast-Enhanced Spectral Mammography (CESM) as a Breast Cancer Risk Factor", Academic Radiology, vol. 27(9), pp. 1234-1240, 2020) folgern, dass Frauen mit erhöhtem BPE ein erhöhtes Risiko für Brustkrebs hatten, unabhängig von anderen potenziellen Risikofaktoren. Das Brustkrebsrisiko bei Frauen mit dichten Brüsten könnte in Kombination mit BPE auf der CESM besser dargestellt werden. Die Verwendung des BPE-Grads bei CESM könnte daher als zusätzliches Instrument zur Beurteilung des Brustkrebsrisikos und für eine vorteilhafte Überwachung wertvoll sein.

Derzeit kann BPE nur visuell auf CEDEM-Bildern beurteilt werden, da es dafür keine softwaregestützten Methoden gibt.

Bei einer visuellen Beurteilung von BPE durch einen Arzt hängen die Ergebnisse stark von diesem Arzt ab (Inter- und Intra-Reader-Variabilität). Berg et al. ("Training Radiologists to Interpret Contrast-enhanced Mammography: Toward a Standardized Lexicon", Journal of Breast Imaging, vol 3 (2), 2021) geben an, dass Radiologen nur eine mäßige Übereinstimmung für BPE zeigen (Mittelwert Kappa = 0,43; Bereich 0,05-0,69). Diese Variabilität verringert den potenziellen Nutzen von BPE als bildgebender Biomarker bei der Gesamtbewertung des Brustkrebsrisikos. Darüber hinaus erfordert die visuelle Beurteilung von BPE Ressourcen (geschulte Radiologen), die möglicherweise nicht immer verfügbar sind.

Für die ce-MRT wurden automatisierte Methoden zur Beurteilung von BPE gezeigt.

Ha et al. ("Fully automated convolutional neural network method for quantification of breast mri fibroglandular tissue and background parenchymal enhancement", J Digit Imaging, vol. 32(141), 2019) führte eine Machbarkeitsstudie durch, um das BPE-Volumen unter Verwendung eines Convolutional Neural Network (CNN) in ce-MRT-Daten zu segmentieren. Eine Einstufung in BPE-Kategorien wurde nicht vorgenommen.

Saha et al. ("Machine learning-based prediction of future breast cancer using algorithmically measured background parenchymal enhancement on high-risk screening MRI", Journal of Magnetic Resonance Imaging, vol. 50(2), pp. 456-464, 2019) haben ein maschinelles Lernmodell trainiert, um BPE aus ce-MRT-Daten zu quantifizieren.

Borkowski et al. ("Fully automatic classification of breast MRI background parenchymal enhancement using a transfer learning approach", Medicine (Baltimore), vol 17(99), pp e21243, 2020) haben eine Methode zur Klassifizierung von BPE in eine der vier Kategorien von ce-MRT-Bildern unter Verwendung eines CNN gezeigt.

Wei et al. ("Fully automatic quantification of fibroglandular tissue and background parenchymal enhancement with accurate implementation for axial and sagittal breast MRI protocols", Medical Physics, vol. 48(1), pp. 238-252, 2021) haben eine Methode untersucht, die zunächst fibroglanduläres Gewebe (FGT) in der T1-gewichteten MRT-Aufnahme segmentiert. Dann werden diese Informationen verwendet, um die volumetrische BPE in diesem interessierenden Volumen durch eine einfache mathematische Formel unter Verwendung der Prä- und Post-Kontrastbilder zu messen.

Nam et al. ("Fully Automatic Assessment of Background Parenchymal Enhancement on Breast MRI Using Machine-Learning Models", Journal of Magnetic Resonance Imaging, vol. 53(3). pp. 818-826, 2021) haben ein lernfähiges Faltungsnetzwerk (CNN) angewendet, um FGT und BPE zu segmentieren, um eine Klassifizierung von BPE-Kategorien vorzunehmen.

Für die kontrastmittelunterstützte 2D-Mammographie oder die kontrastmittelunterstützte 3D-Brust-Tomosynthese wurden bisher keine automatisierten Verfahren zur Beurteilung von BPE vorgestellt.

WO 2022/003656 A1 offenbart ein Verfahren zur Quantifizierung des BPE, bei dem zur visuellen Beurteilung eine "additional contrast concentration map" erstellt wird, und darauf basierend zusammenfassende Statistiken erstellt werden, wie beispielsweise der Durchschnitt, die Standardabweichung oder die maximal erhöhte Kontrastmittelkonzentration in dichtem Gewebe.

Es ist eine Aufgabe der vorliegenden Erfindung, ein verbessertes Verfahren und ein entsprechendes System zur Bestimmung des BPE in einer kontrastmittelverstärkten Röntgenuntersuchung einer Brust anzugeben, mit dem die oben beschriebenen Nachteile vermieden werden und insbesondere BPE auf kontrastmittelverstärkten Mammographieaufnahmen, z.B. CEDEM-Bildern oder CEDET-Bildern zu beurteilen, da BPE als ein wertvoller Biomarker bei der Gesamtbewertung des Brustkrebsrisikos dienen kann.

Diese Aufgabe wird durch ein Verfahren gemäß Patentanspruch 1, ein System gemäß Patentanspruch 11, eine Steuereinrichtung gemäß Patentanspruch 12 sowie durch ein Mammographiesystem gemäß Patentanspruch 13 gelöst.

Das erfindungsgemäße Verfahren zur Bestimmung des BPE in einer kontrastmittelverstärkten Röntgenuntersuchung einer Brust umfasst die folgenden Schritte:

- Bereitstellen von Bildern der Röntgenuntersuchung, die nach Verabreichung eines Kontrastmittels aufgenommen worden sind, die Bilder umfassend zumindest ein LE-Bild, welches mit einer vorgegebenen niedrigen Röntgenenergie aufgenommen worden ist und ein HE-Bild, welches mit einer vorgegebenen hohen Röntgenenergie aufgenommen worden ist,
- Erstellen eines (bevorzugt kalibrierten) Jodbildes aus dem LE-Bild und dem HE-Bild,
- Berechnen eines volumetrischen BPE als Summe aller Bildpunkt-Werte im Jodbild, normiert auf ein Volumen, für die die folgenden Bedingungen zutreffen:

  i) es befindet sich dort im Jodbild eine Jod-Anreicherung,
  ii) es befindet sich dort fibroglanduläres Gewebe,

- Ermitteln von BPE-Ergebnisdaten basierend auf dem Jodbild und dem volumetrischen BPE,
- Ausgabe der BPE-Ergebnisdaten.

Bei einer kontrastmittelverstärkten Röntgenuntersuchung einer Brust erhält man nach dem Verfahren mindestens zwei Bilder: Das LE-Bild und das HE-Bild. Es handelt sich also bei der Röntgenuntersuchung um eine Dual-, oder eine Multi-Energie-Röntgenuntersuchung, bei der Aufnahmen mit zwei oder mehreren unterschiedlichen Röntgenenergien durchgeführt werden. Bevorzugt handelt es sich dabei um eine CEDEM-Untersuchung (s.o. "Contrast-Enhanced Dual Energy Mammography") oder eine CEDET-Untersuchung ("Contrast-Enhanced Dual Energy Tomosynthese"). Vor der Untersuchung wird ein Kontrastmittel verabreicht, insbesondere intravenös.

Das LE-Bild wird typischerweise als erstes Bild bei einer niedrigen Röntgenenergie aufgenommen (LE: "low energy"). Nach einem kurzen Zeitintervall, während sich jedoch die Brust noch in derselben Kompressionsphase wie zuvor befindet, wird dann das HE-Bild bei einer hohen Röntgenenergie aufgenommen (HE: "high energy"). Es können durchaus weitere Bilder bei weiteren Energien aufgenommen werden, wobei sich die Kompressionsphase der Brust nicht ändern sollte. Es ergeben sich (zumindest) das LE-Bild und das HE-Bild, die bevorzugt in einem Ortsraum rekonstruiert werden, wenn sie nicht bereits Ortsinformationen wiedergeben (z.B. bei einer Tomographie). Es handelt sich bei den beiden Bildern also um Multi- oder Dual-Energie Aufnahmen mit einer höheren (HE-Bild) und einer niedrigeren Energie (LE-Bild).

Jedes dieser Bilder wird aus Bildelementen (Pixel oder Voxel) gebildet, die zumindest einen Bildwert aufweisen. Bei Graustufenbildern liegt dieser Bildwert zumeist zwischen 0 und 255, 1024 oder auch einer sehr viel höheren natürlichen Zahl, bei bunten Bildern können auch drei Bildwerte pro Bildelement vorliegen, welche die Grundfarben wiedergeben.

Jedes Bildelement liegt an einer individuellen Bildposition (Bildkoordinate). Entsprechende Bildelemente entsprechen demselben Punkt des Objekts. Bei Bildern, die dieselbe Größe aufweisen und in denen das Objekt in einer identischen Position abgebildet wurde, haben entsprechende Bildelemente dieselbe Bildkoordinate.

Aus den beiden Bildern (also LE-Bild und HE-Bild und ggf. weiteren Bildern einer Multi-Energie Aufnahme) wird dann das Jodbild erstellt. Dabei handelt es sich um ein synthetisches Bild, welches die Jodkonzentration wiedergeben soll. Die Erstellung des Jodbildes ist im Stand der Technik bekannt und erfolgt häufig nach der Formel

$$\text{Jodbild} = \ln(\text{HE-Bild}) - w \cdot \ln(\text{LE-Bild}),$$

mit dem natürlichen Logarithmus ln und einem vorgegebenen Wichtungsfaktor w. Es werden dabei die einzelnen Bildwerte des Jodbildes aus den entsprechenden Bildwerten des LE-Bildes und HE-Bildes berechnet. Bevorzugt wird ein kalibriertes Jodbild berechnet, was im Stand der Technik typischerweise bei der Berechnung eines Jodbildes erfolgt.

Unter idealen Bedingungen (monochromatische Röntgenquelle, keine Streustrahlung etc.) besteht ein linearer Zusammenhang zwischen der Signalintensität in einem Pixel im Jodbild und der Jodkonzentration in dem kegelförmigen Bereich, der von der vom der Röntgenquelle und dem entsprechenden Aufnahmebereich des Detektors (z.B. Detektorpixel) aufgespannt wird. Unter realistischen Bedingungen ist dieser lineare Zusammenhang immer noch eine gute Annäherung. Es gibt mehrere Methoden, die auf eine Quantifizierung der Jodkonzentration in einem CEDEM-Bild abzielen, wie z. B. die Verwendung eines Kompartimentmodells (s. Laidevant et al (2010), "Compositional breast imaging using a dual-energy mammography protocol", Medical Physics, vol 37(1), pp. 164-174). Auch für CEDET-Bilder sind Quantifizierungsverfahren bekannt (s. Michielsen et al (2020), "Iodine quantification in limited angle tomography", Medical Physics., vol. 47(10)).

BPE bezieht sich auf die bildliche Verstärkung von fibroglandulärem Gewebe (FGT) nach intravenöser Verabreichung eines Kontrastmittels. Für dieses Gewebe wird typischerweise auch im Deutschen der englische Ausdruck "fibroglandular tissue" verwendet und im Folgenden daher die Abkürzung "FGT" zum Hinweis auf das fibroglanduläre Gewebe benutzt. Bei einer kontrastmittelverstärkten Dual- oder Multi-Energie-Röntgenuntersuchung, insbesondere bei einer (Jod-)Kontrastunterstützten Röntgenuntersuchung, ist FGT am besten im LE-Bild sichtbar, während BPE am besten aus dem Jodbild entnommen werden kann.

Es kann somit zur Berechnung des volumetrischen BPE (vBPE) einfach über alle Bildelemente des Jodbildes hinweg geschaut werden, ob dort eine Jodanreicherung zusammen mit einem FGT vorliegt. Diesbezüglich kann vorteilhaft mit Masken gearbeitet werden, die auch weitere Informationen für zusätzliche Auswertungen bereithalten können.

Der Schritt der Berechnung des volumetrischen BPE kann insbesondere die folgenden Schritte umfassen:

- Erstellen einer FGT-Maske, in dem eine Klassifizierung für jedes Bildelement im LE-Bild oder einer gewichteten Linearkombination des LE-Bildes und einer Anzahl weiterer spektraler Bilder durchgeführt wird, ob es sich im Bildelement um fibroglanduläres Gewebe handelt bzw. ob das Bildelement fibroglanduläres Gewebe darstellt, wobei im positiven Fall eine entsprechende Bildposition in der FGT-Maske mit einem FGT-Marker markiert wird,
- Erstellen einer FGT-E-Maske, in dem an den Bildpositionen im Jodbild, deren Entsprechungen in der FGT-Karte mit einem FGT-Marker markiert sind, eine Klassifizierung stattfindet, ob dort eine Jod-Anreicherung vorliegt, wobei im positiven Fall eine entsprechende Bildposition in der FGT-E-Maske mit einem FGTE-Marker versehen wird,
- Berechnen eines volumetrischen BPE als Summe aller Bildpunkt-Werte im Jodbild bei denen an der entsprechenden Stelle der FGT-E-Maske ein FGTE-Marker vorliegt.

Zusätzlich zum Jodbild wird also eine FGT-Maske aus dem LE-Bild berechnet. Grundsätzliche Prinzipien zur automatischen Erkennung von FGT sind im Stand der Technik bekannt. Da ein LE-CEDEM-Bild einer normalen Mammographie ähnelt (s. z.B. Gennaro et al. "Quantitative Breast Density in Contrast-Enhanced Mammography", J. Clin. Med. 2021, 10(15), 3309), kann z.B. das von Fieselmann et al. (in "Volumetric breast density measurement for personalized screening: accuracy, reproducibility, consistency, and agreement with visual assessment", Journal of Medical Imaging, vol. 6(3) pp. 031406, 2019) beschriebene Verfahren zur Erstellung einer Brustdichtekarte verwendet werden. Methoden zur Bestimmung der FGT aus einer Tomosynthese-Untersuchung sind ebenfalls bekannt (s. z.B. Ekpo and McEntee, "Measurement of breast density with digital breast tomosynthesis-a systematic review", British Journal of Radiology, vol 87(1043), 2014).

Bevorzugt wird aus dem erkannten FGT die FGT-Maske berechnet. Dazu wird für jedes Pixel im LE-Bild (automatisch) entschieden, ob dieses FGT zeigt oder nicht. Dies kann in der Praxis z.B. durch Schwellenwertbildung einer Brustdichtekarte mit einem vorbestimmten Grenzwert erfolgen. Ein sinnvoller Grenzwert wird zunächst ermittelt und dieses dann auf alle Pixel der Brustdichtekarte angewandt. Liegt ein Bildwert über dem Grenzwert, wird in der FGT-Maske an der entsprechenden Stelle ein FGT-Marker gesetzt, z.B. "1", der Anzeigt, dass dort FGT vorliegt. Ansonsten wird kein oder ein anderer Wert, z.B. "0", gesetzt. Der FGT-Marker kann eine beliebige Form bzw. einem beliebigen Wert haben, sofern er als Marker für FGT im LE-Bild verwendbar ist. Beispielsweise kann die FGT-Maske ein Bild sein, welches das gleiche Bildformat wie das LE-Bild aufweist und an den Bildkoordinaten, an denen im LE-Bild FGT vorliegt, FGT-Marker aufweist.

Liegen Jodbild und FGT-Maske vor, kann die FGT-E-Maske erstellt werden. Dazu wird nach der Vorgabe der FGT-Maske (also nur dort wo fibroglanduläres Gewebe vorliegt, also ein FGT-Marker vorhanden ist) an der gleichen Stelle im Jodbild geschaut, ob an dieser Stelle eine Jod-Anreicherung vorliegt. Dazu kann z.B. der Bildwert eines entsprechenden Pixels mit einem vorbestimmten Grenzwert verglichen werden und in dem Falle, in dem der Bildwert größer ist als der Grenzwert, eine Jod-Anreicherung angenommen werden. Dies wird dann für alle Pixel der FGT-Maske durchgeführt, die FGT anzeigen (z.B. den Wert "1" haben). Wenn das Jodbild kalibriert ist, kann der Grenzwert ein bestimmter Wert sein (z. B. abhängig von der Brustdicke). Der Grenzwert kann aber auch anhand des Signalhistogramms des Jodbildes bestimmt werden (z. B. ein Wert relativ zum Bereich 5%-95% Anteil der Intensitäten). Beispielsweise kann die FGT-E-Maske ein Bild sein, welches das gleiche Bildformat wie das Jodbild aufweist und an den Bildkoordinaten, an denen im LE-Bild FGT vorliegt (FGT-Marker) und im Jodbild eine Jod Anreicherung aufweist, FGTE-Marker aufweist.

Die Klassifizierung ist bevorzugt binär, so dass die Bildposition in der FGT-E-Maske, an der im Jodbild eine Jod-Anreicherung vorliegt, mit einem FGTE-Marker versehen wird, z.B. mit dem Wert 1. Die übrigen Bereiche der FGT-E-Maske können Werte aufweisen, die angeben, dass keine Jod-Anreicherung vorliegt oder die FGT-Maske dort kein FGT anzeigt, z.B. den Wert "0". Der Begriff "FGTE-Marker wird verwendet, um diesen Marker vom FGT-Marker der FGT-Maske zu unterscheiden. Auch die Klassifizierung der FGT-Karte ist bevorzugt binär.

Die FGT-Karte und/oder die FGT-E-Karte können in Form von Bildern vorliegen, die bevorzugt dieselbe Größe bzw. dasselbe Format wie die LE-Bilder und/oder das Jodbild haben. Ein Vergleich Bildpunkt für Bildpunkt von entsprechenden räumlichen Positionen ist dann besonders einfach zu realisieren. Bevorzugt entspricht jeder Bildpunkt des Jodbildes einem Bildpunkt des jeweiligen LE-Bildes und HE-Bildes. Dann ist besonders vorteilhaft, wenn jeder Bildpunkt der FGT-Maske (besetzt mit einem FGT-Marker oder keinem Marker) einem Bildpunkt des LE-Bildes (und damit auch des Jodbildes) entspricht (mit entsprechenden Werten zur Klassifikation) und jeder Bildpunkt der FGT-E-Maske einem Bildpunkt des Jodbildes entspricht (mit entsprechenden FGTE-Markern zur Klassifikation).

Liegt die FGT-E-Maske vor, dann kann das volumetrische BPE ("vBPE") unter Verwendung dieser FGT-E-Maske und des Jodbildes berechnet werden. Dabei wird die Summe aller Bildpunkt-Werte im Jodbild berechnet, bei denen an der entsprechenden Stelle der FGT-E-Maske ein FGTE-Marker vorliegt. Es ist dabei vorgesehen, diese Summe S auf das Volumen zu normieren, also im Grunde als vBPE eine BPE-Dichte S zu berechnen, so dass z.B. das volumetrische BPE pro Kubikzentimeter oder pro Milliliter vorliegt. Damit können Brüste unterschiedlicher Größe gut verglichen werden bzw. vergleichbare BPE-Grenzwerte angegeben werden. Das volumetrische BPE hat gegenüber einem flächig ermittelten BPE Vorteile, da es das Brustkrebsrisiko genauer angibt.

Die Einheit des vBPE ist bevorzugt dieselbe, die für das Jodbild verwendet wird. Falls das Jodbild kalibriert ist, ist eine bevorzugte Einheit "mg Jod / ml". Wenn das Jodbild nicht kalibriert ist, kann der unkalibrierte vBPE* bestimmt werden. Die Werte vBPE und vBPE* können durchaus zur Berechnung weiterer Metriken verwendet werden Im Folgenden sind mit dem volumetrischen BPE (vBPE) beide Fälle umfasst.

Danach können BPE-Ergebnisdaten basierend auf dem Jodbild und dem volumetrischen BPE ermittelt werden. Diese können im Grunde bereits das volumetrische BPE sein. Als BPE-Ergebnisdaten kann aber auch die vorgenannte BPE-Dichte berechnet werden (wenn das vBPE noch keine Dichte Darstellt). Es kann auch in den BPE-Ergebnisdaten ein BPE-Bild enthalten sein, welches die Bildwerte der Jodkarte an den Positionen der FGTE-Marker der FGT-E-Karte wiedergibt.

Es ist zu beachten, dass in der Regel die Konzentration von BPE in vier Kategorien eingeteilt wird: "minimal", "leicht", "mäßig" und "ausgeprägt". Auch wenn diese Kategorisierung mittels eines Flächenanteils in dem BPE-Bild durchgeführt werden kann, ist es vorteilhaft, sowohl das Volumen als auch die Intensität oder die Verteilung und Morphologie zu berücksichtigen. Diese Kategorisierung kann ebenfalls basierend auf dem volumetrischen BPE und der Jodkarte bzw. mittels des BPE-Bildes vorgenommen werden und in den BPE-Ergebnisdaten enthalten sein.

Diese BPE-Ergebnisdaten werden dann ausgegeben, z.B. auf einem Bildschirm, so dass ein Nutzer sie betrachten kann. Sie können aber auch einfach abgespeichert werden, bis sich ein Mediziner damit befassen kann.

Ein erfindungsgemäßes System zur Bestimmung des BPE in einer kontrastmittelverstärkten Röntgenuntersuchung einer Brust gemäß einem Verfahren nach einem der vorangehenden Ansprüche umfasst die folgenden Komponenten:

- eine Datenschnittstelle ausgelegt zum Empfang von Bildern der Röntgenuntersuchung, die nach Verabreichung eines Kontrastmittels aufgenommen worden sind, die Bilder umfassend zumindest ein LE-Bild, welches mit einer vorgegebenen niedrigen Röntgenenergie aufgenommen worden ist und ein HE-Bild, welches mit einer vorgegebenen hohen Röntgenenergie aufgenommen worden ist,
- eine Jodbild-Einheit ausgelegt zum Erstellen eines Jodbildes aus dem LE-Bild und dem HE-Bild,
- eine BPE-Einheit ausgelegt zum Berechnen eines volumetrischen BPE als Summe aller Bildpunkt-Werte im Jodbild (J), normiert auf ein Volumen, für die die folgenden Bedingungen zutreffen:

    i) es befindet sich dort im Jodbild eine Jod-Anreicherung,
    ii) es befindet sich dort fibroglanduläres Gewebe,

und ausgelegt zum Ermitteln von BPE-Ergebnisdaten basierend auf dem Jodbild und dem volumetrischen BPE,
- eine Datenschnittstelle ausgelegt zum Ausgeben der BPE-Ergebnisdaten.

Zur vorteilhaften Berechnung des vBPE kann das System, insbesondere dessen BPE-Einheit, die folgenden Einheiten umfassen:

- eine FGT-Einheit ausgelegt zum Erstellen einer FGT-Maske, in dem eine Klassifizierung für jedes Bildelement im LE-Bild oder einer gewichteten Linearkombination des LE-Bildes und einer Anzahl weiterer spektraler Bilder durchgeführt wird, ob das Bildelement fibroglanduläres Gewebe darstellt bzw. ob es sich im Bildelement um fibroglanduläres Gewebe handelt, wobei im positiven Fall eine entsprechende Bildposition in der FGT-Maske mit einem FGT-Marker markiert wird,
- eine FGTE-Einheit ausgelegt zum Erstellen einer FGT-E-Maske, in dem an den Bildpositionen im Jodbild, deren Entsprechungen in der FGT-Karte mit einem FGT-Marker markiert sind, eine Klassifizierung stattfindet, ob dort eine Jod-Anreicherung vorliegt, wobei im positiven Fall eine entsprechende Bildposition in der FGT-E-Maske mit einem FGTE-Marker versehen wird.

wobei die BPE-Einheit dazu ausgelegt ist, den volumetrischen BPE als Summe aller Bildpunkt-Werte im Jodbild, normiert auf ein Volumen, bei denen an der entsprechenden Stelle der FGT-E-Maske ein FGTE-Marker vorliegt, zu berechnen.

Die Komponenten arbeiten gemäß den vorangehend beschriebenen Schritten des Verfahrens. Damit kann eine leserunabhängige automatisierte Bewertung von BPE zur Verfügung gestellt werden.

Eine erfindungsgemäße Steuereinrichtung ist zur Steuerung eines Mammographiesystems ausgelegt. Solche Steuereinrichtungen sind im Grunde im Stand der Technik bekannt. Allerdings umfasst eine erfindungsgemäße Steuereinrichtung ein erfindungsgemäßes System.

Ein erfindungsgemäßes Mammographiesystem umfasst eine erfindungsgemäße Steuereinrichtung.

Die Erfindung kann insbesondere in Form einer Rechnereinheit, insbesondere in einer Steuereinrichtung für ein Mammographiesystem, mit geeigneter Software realisiert sein. Die Rechnereinheit kann z.B. hierzu einen oder mehrere zusammenarbeitende Mikroprozessoren oder dergleichen aufweisen. Insbesondere kann sie in Form von geeigneten Softwareprogrammteilen in der Rechnereinheit realisiert sein. Eine weitgehend softwaremäßige Realisierung hat den Vorteil, dass auch schon bisher verwendete Rechnereinheiten auf einfache Weise durch ein Software- bzw. Firmware-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Rechnereinheit ladbar ist, mit Programmabschnitten, um alle Schritte des erfindungsgemäßen Verfahrens auszuführen, wenn das Programm in der Rechnereinheit ausgeführt wird. Ein solches Computerprogrammprodukt kann neben dem Computerprogramm gegebenenfalls zusätzliche Bestandteile wie z.B. eine Dokumentation und/oder zusätzliche Komponenten, auch Hardware-Komponenten, wie z. B. Hardware-Schlüssel (Dongles etc.) zur Nutzung der Software, umfassen. Zum Transport zur Rechnereinheit und/oder zur Speicherung an oder in der Rechnereinheit kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Ein bevorzugtes Verfahren, umfasst die folgenden zusätzlichen Schritte:

- Zählen der Anzahl von FGT-Markern der FGT-Maske (also die Anzahl der Bildpunkte des LE-Bildes, an denen FGT vorliegt) als X und der Anzahl von FGTE-Markern der FGT-E-Maske (also die Anzahl der Bildpunkte, wo im LE-Bild FGT vorliegt und zusätzlich im Jodbild eine Jod-Anreicherung vorliegt) als XE,
- Berechnen des relativen BPE ("rBPE") mit dem Quotienten rBPE = XE / X.

Der rBPE ist der jodanreichernde relative, prozentuale Anteil des FGT. Die Berechnung kann als relatives Maß und als Zusatzinformation für einen Radiologen hilfreich sein.

In der Praxis entfaltet die Erfindung einen großen Nutzen, wenn sie neben der Durchführung einer automatisierten Bestimmung des BPE-Volumens zusätzlich noch eine automatisierte Klassifikation von BPE im Jodbild durchführt.

Gemäß einem bevorzugten Verfahren wird daher zusätzlich eine automatisierte Klassifikation von BPE-Ergebnisdaten im Jodbild durchgeführt. Bevorzugt wird dazu eine ortsabhängige Klassifikation durch Anwendung einer Zuordnungs-Funktion auf Bildelementen des Jodbilds ermittelt, insbesondere einer Zuordnungs-Funktion, die Intervallen eines kontinuierlichen Wertebereichs konkrete Bezeichnungen zuweist. Hier sind vier Bezeichnungen, die vier unterschiedliche Stärkegrade angeben, z.B. in die Kategorien "minimal", "leicht", "mäßig" und "ausgeprägt" bevorzugt. Es kann aber auch ein anderer Wertebereich gewählt werden.

Im Vergleich zum volumetrischen BPE, das als kontinuierliche Größe eine gute Beschreibung des BPE ermöglicht (z.B. die in Instrumente zur Risikobewertung von Brustkrebs aufgenommen werden kann), ist in der klinischen Praxis oftmals besagte Klassifizierung von BPE vorteilhaft. Eine Klassifizierung von BPE, insbesondere aus CEDEM/T-Untersuchungsdaten, kann vorteilhaft basierend auf dem relativen BPE (rBPE) und/oder dem volumetrischen BPE (vBPE) vorgenommen werden, wobei dabei kontinuierlichen Werten diskrete Kategorien zuordnet werden, z.B. in dem überprüft wird, ob der Wert in einem Werteintervall liegt, der eine Klassifizierung repräsentiert. Die Werteintervalle können dabei durch Kalibrierung mit Labels von Radiologen auf einem Datensatz von CEDEM/T-Bildern erhalten werden, für die die vBPE-Werte oder rBPE-Werte berechnet wurden.

Auch ist es von Vorteil, wenn die Ergebnisse grafisch Angezeigt werden, insbesondere zur Erleichterung der Interpretation durch einen Mediziner. Insbesondere sollte eine solche Anzeige zusammen in Form einer Kombination der Ergebnisse pro Ansicht und pro Brust erfolgen, um die BPE-Beurteilung in den klinischen Arbeitsablauf optimal zu integrieren.

Gemäß einem bevorzugten Verfahren erfolgt im Rahmen der Ausgabe der BPE-Ergebnisdaten eine grafische Anzeige des Jodbildes oder eines aus dem Jodbild abgeleiteten Bildes. Alternativ oder zusätzlich erfolgt bevorzugt die Anzeige des volumetrischen BPE und/oder eines Wertes rBPE und besonders bevorzugt eine Kombination von Ergebnissen per Ansicht und per Brust.

Es ist in der Praxis von Vorteil, wenn pro Patientin am Schluss ein Wert vorhanden ist, z.B. 4 Aufnahmen, je zwei von jeder Brust, und durch Kombination (z.B. Mittelung) am Schluss ein Wert, z.B. ein Maximalwert, für das BPE vorliegt. Auch können Klassifizierungsergebnisse zusammen mit einer Information visualisiert werden, wie nahe die Kategorie an einer benachbarten Kategorie liegt. In den BPE-Ergebnisdaten können auch Unterschiede von BPE-Werten, insbesondere von vBPE oder rBPE enthalten, sein, wobei die Unterschiede bevorzugt zeitlicher Natur sind (vor/nach der Behandlung) oder Unterschiede der Bewertung zwischen den beiden Brüsten (Asymmetrien) sind.

Gemäß einem bevorzugten Verfahren wird zum Erstellen der FGT-Maske zunächst eine Brustdichtekarte erstellt oder bereitgestellt und durch Schwellenwertbildung der Brustdichtekarte mit einem vorbestimmten Schwellenwert die FGT-Maske erstellt.

Es ist bekannt, dass BPE und mammografische Brustdichte (die durch den FGT wiedergegeben wird) stark korrelieren. Eine Nichtübereinstimmung des BPE- und Brustdichtemusters im Bild könnte ein Indikator für einen relevanten klinischen Zustand sein.

Gemäß einem bevorzugten Verfahren erfolgt eine automatisierte Erkennung der Abweichung zwischen einer lokalen mammographischen Brustdichte und den BPE-Ergebnisdaten basierend auf dem LE-Bild und dem Jodbild. Bei Nichtübereinstimmung des Musters des BPE und der Brustdichte ein Indikator für einen relevanten klinischen Zustand generiert wird.

Mit den folgenden Schritten kann man eine Diskrepanz zwischen BPE und Brustdichte vorteilhaft erkennen:

- Berechnen einer Brustdichtekarte aus dem LE-Bild oder einer Linearkombination von LE- und HE-Bild (oder weiterer spektraler Bilder z.B. aus einer Triple Energy Akquisition), z.B. unter Verwendung einer von Fieselmann et al. beschriebenen Methode ("Volumetric breast density measurement for personalized screening: accuracy, reproducibility, consistency, and agreement with visual assessment", Journal of Medical Imaging, vol. 6(3) pp. 031406, 2019),
- Vergleichen der Intensitätswerte der Bildelemente (Pixel bzw. Voxel) der Brustdichtekarte mit den entsprechenden Bildelementen im Jodbild, wobei der verglichene Bildbereich bevorzugt durch die FGT-Maske und/oder die FGT-E-Maske vorgegeben wird (bzw. durch diese eingeschränkt wird), unter Bildung einer Anzahl von Abweichungswerten, wobei in vorgegebenen Sub-Bereichen, insbesondere einzelne Pixel oder Pixelgruppen, z.B. quadratische ROIs (vorteilhaft sind Seitenlängen um die 3 cm), mittlere Abweichungswerte berechnet werden,
- Ermitteln, ob die Abweichungswerte lokal oder global außerhalb eines vorgegebenen Wertebereichs liegen, insbesondere durch Anwendung einer Korrelationsanalyse.

Eine lokale Fehlanpassung wird erkannt, wenn die beiden Mittelwerte der Ergebnisse der beiden Brüste vom globalen Verhalten abweichen z.B. bestimmt durch Anwendung einer Korrelationsanalyse der gesamten Bilder. Die Menge nicht übereinstimmender ROIs kann quantifiziert werden (z.B. relative Menge nicht übereinstimmender ROIs).

BPE kann auf verschiedene Arten quantitativ analysiert werden. Eine vorteilhafte Analyse betrifft eine globale Charakterisierung (räumliche Verteilung), oder genauer: Ob die BPE lokalisiert oder homogen im Bild verteilt ist.

Gemäß einem bevorzugten Verfahren erfolgt eine automatisierte Analyse der räumlichen Verteilung, Morphologie und Textur von BPE im Jodbild. Ein möglicher Quantifizierungsansatz kann auf der sequentiellen Anwendung morphologischer Operatoren beruhen, wie in der Schrift EP 3073924 A1 für die Quantifizierung des Maskierungsrisikos von FGT in Mammogrammen beschrieben. Es ist bevorzugt, dass abweichend von dieser Methode das Verfahren auf die FGT-E-Maske und/oder das Jodbild angewendet und nicht auf die Brustdichtekarte. Es wird also bevorzugt ermittelt, ob die BPE lokalisiert oder homogen im Jodbild verteilt ist, insbesondere wobei morphologische Operatoren sequentiell auf das Jodbild oder die auf die FGT-E-Maske angewendet werden.

Eine weitere vorteilhafte Analyse betrifft eine lokale Charakterisierung, d.h. wie die BPE auf lokaler Ebene erscheinen (Morphologie und Textur). In der Literatur sind zahlreiche Methoden zur Analyse des lokalen Erscheinungsbildes bekannt, z.B. Verwenden von Haralick-Texturfunktionen. Diese Methoden werden auf die FGT-E-Maske und/oder das Jodbild angewendet. Es wird also bevorzugt die Morphologie und Textur der BPE ermittelt, insbesondere unter Verwendung von Haralick-Texturfunktionen, welche auf die FGT-E-Maske und/oder das Jodbild angewendet werden. Dabei werden insbesondere quantitative bildgebende Biomarker aus dem Jodbild, der FGT-E-Maske, dem LE-Bild, dem HE-Bild und/oder davon abgeleiteten Bildern oder Masken extrahiert.

Durch die Verwendung der globalen und/oder lokalen Charakterisierung können quantitative bildgebende Biomarker aus den CEDEM/T-Untersuchungsdaten extrahiert werden. Diese Biomarker können zur Vorhersage von individuellen Merkmalen von Patienten, wie etwa ein Ansprechen auf eine Brustkrebsbehandlung, verwendet werden.

Aus Studien, die auf ce-MRT basieren, ist bekannt, dass BPE-Veränderungen während einer neoadjuvanten Chemotherapie (NAC) ein bildgebender Biomarker für das Ansprechen auf die Behandlung sein können. Eine frühe Reduktion von BPE in der kontralateralen Brust während einer NAC kann ein früher Prädiktor für den Verlust des Tumoransprechens sein. Somit kann diese Reduktion von BPE als Biomarker für das Ansprechen auf die Behandlung verwendet werden, insbesondere bei Frauen mit Brustkrebs im Stadium 3 oder 4 und bei HER2-negativem Brustkrebs (s. Rella et al. (2020), Association between background parenchymal enhancement and tumor response in patients with breast cancer receiving neoadjuvant chemotherapy, Diagnostic Interventional Imaging, vol. 101(10), pp. 649-655,).

Zusätzlich zur ce-MRT oder wenn ce-MRT nicht verfügbar ist, kann die BPE-Veränderung im Laufe der Zeit anhand von CEDEM/T-Untersuchungen bestimmt werden, um das Ansprechen auf die Behandlung vorherzusagen oder den Behandlungserfolg zu überwachen.

Als quantitativer bildgebender Biomarker kann die relative Veränderung von vBPE berechnet werden. Ebenso könnte eine Änderung der räumlichen Verteilung, Morphologie und Textur von BPE zwischen Zeitpunkten basierend auf den numerischen Werten von Merkmalen, die diese Eigenschaften beschreiben, quantifiziert werden.

Gemäß einem bevorzugten Verfahren wird eine automatisierte Längsschnittbewertung von BPE basierend auf dem Jodbild durchgeführt. Es ist dabei bevorzugt, dass das Verfahren mit LE-Bildern und HE-Bildern mehrerer Röntgenuntersuchungen der gleichen Person durchgeführt wird und die relative Veränderung des volumetrischen BPE über die unterschiedlichen Röntgenuntersuchungen hinweg berechnet wird und oder eine Änderung der räumlichen Verteilung, der Morphologie und/oder der Textur von BPE berechnet wird.

Es sollte auf signifikante Unterschiede des BPE-Musters in der linken und rechten Brust geachtet werden. Für eine BPE-Beurteilung aus CEDEM/T-Untersuchungsdaten ist es vorteilhaft jede Asymmetrie zu quantifizieren und zu berücksichtigen. Wang et al. ("Computerized Detection of Breast Tissue Asymmetry Depicted on Bilateral Mammograms: A Preliminary Study of Breast Risk Stratification", Academic Radiology, vol 17(10), pp. 1234-1241, 2010) beschreibt eine Methode zur Quantifizierung der Asymmetrie in Röntgenuntersuchungen der Brust, die beispielsweise auch auf das Jodbild angewendet werden könnte.

Gemäß einem bevorzugten Verfahren wird basierend auf LE-Bildern und HE-Bildern einer bilateralen mammographischen Untersuchung (Aufnahme beider Brüste) eine automatisierte Bewertung der Symmetrie basierend auf dem Jodbild einer aufgenommenen rechten Brust und einer aufgenommenen linken Brust durchgeführt.

Gemäß einem bevorzugten Verfahren erfolgte die Röntgenuntersuchung mittels eines spektralen Mehrenergie-Bildgebungsverfahrens (z.B. Dreifachenergie), einschließlich einer bevorzugten Verwendung von Mehrschichtdetektoren für die Bildaufnahme. Das Jodbild und/oder die FGT-E-Maske und/oder insbesondere die FGT-Maske wird dabei bevorzugt mittels mindestens eines weiteren Bildes generiert, welches bei einer anderen Energie als das LE-Bild und das HE-Bild aufgenommen wurde.

Die vorgeschlagenen Bewertungsschritte können auch in einzelnen oder kombinierten Bildern durchgeführt werden.

In einer weiteren alternativen Ausgestaltung kann das vorgeschlagene Verfahren auf die kontrastmittelverstärkte Brust-CT als weiteres Röntgenbildgebungs- und vollständiges 3D-Akquisitionsverfahren übertragen werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1 eine grob schematische Darstellung eines bevorzugten Mammographiesystems mit einem bevorzugten System,

Figur 2 einen Ablaufplan für einen möglichen Ablauf eines erfindungsgemäßen Verfahrens,

Figur 3 ein Beispiel für aufgenommene LE-Bilder und HE-Bilder,

Figur 4 ein Beispiel für Jodbilder,

Figur 5 ein Beispiel für FGT-Masken und FGT-E-Masken,

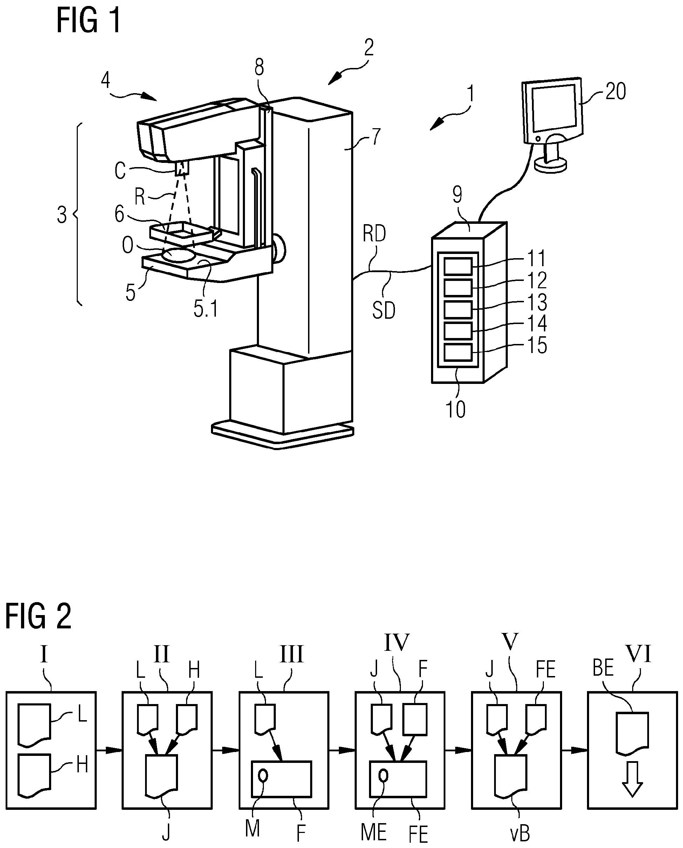

In Figur 1 ist beispielhaft und grob schematisch ein Mammographiesystem 1 in Form eines Tomosynthesesystems 1 gezeigt. Relative Richtungsangaben wie "oben", "unten" etc. beziehen sich auf ein bestimmungsgemäß für den Betrieb aufgestelltes Tomosynthesesystem 1. Das Tomosynthesesystem 1 umfasst ein Tomosynthesegerät 2 und eine Steuereinrichtung 9.

Das Tomosynthesegerät 2 weist eine Standsäule 7 und eine Quelle-Detektor-Anordnung 3 auf, die wiederum eine Röntgenstrahler 4 und einen Detektor 5 mit einer Detektorfläche 5.1 umfassen. Die Standsäule 7 steht im Betrieb auf dem Untergrund. Mit ihr ist die Quelle-Detektor-Anordnung 3 verschiebbar verbunden, sodass die Höhe der Detektorfläche 5.1, also der Abstand zum Untergrund, auf eine Brusthöhe einer Patientin eingestellt werden kann. Das Tomosynthesesystem 1 ist hier beispielhaft für CEDEM bzw. CEDET Aufnahmen ausgelegt und kann mit zwei Röntgenenergien messen, so dass ein LE-Bild und ein HE-Bild aufgenommen werden kann.

Eine Brust O der Patientin (hier schematisch dargestellt) liegt als Untersuchungsobjekt O für eine Untersuchung oberseitig auf der Detektorfläche 5.1 auf. Über der Brust O und der Detektorfläche 5.1 ist eine Platte 6 angeordnet, die verschiebbar mit der Quelle-Detektor-Anordnung 3 verbunden ist. Für die Untersuchung wird die Brust O komprimiert und zugleich fixiert, indem die Platte 6 auf sie herabgesenkt wird, sodass auf die Brust O zwischen Platte 6 und Detektorfläche 5.1 ein Druck ausgeübt wird.

Der Röntgenstrahler 4 ist dem Detektor 5 gegenüberliegend so angeordnet und ausgebildet, dass der Detektor 5 von ihm emittierte Röntgenstrahlung R erfasst, nachdem zumindest ein Teil der Röntgenstrahlung R die Brust O der Patientin durchdrungen hat. Dabei ist der Röntgenstrahler 4 relativ zum Detektor 5 mittels eines Dreharms 8 in einem Bereich von ± 50° um eine Grundstellung schwenkbar, in der sie senkrecht über der Detektorfläche 5.1 steht. Der aufzunehmende Ausschnitt kann mittels eines Kollimators C, der auch Filter C enthalten kann, vorgegeben bzw. eingeschränkt werden.

Die Steuereinrichtung 9 erhält die Rohdaten RD der Messung und sendet Steuerdaten SD an das Tomosynthesegerät 2 mittels einer Datenschnittstelle. Sie ist mit einem Terminal 20 verbunden, über den ein Benutzer dem Tomosynthesesystem 1 Befehle mitteilen oder Messergebnisse abrufen kann. Die Steuereinrichtung 9 kann in demselben Raum wie das Tomosynthesegerät 2 angeordnet sein, es kann sich aber auch in einem angrenzenden Kontrollraum oder in einer noch weiteren räumlichen Entfernung befinden.

Das erfindungsgemäße System 10 zur Bestimmung des BPE in einer kontrastmittelverstärkten Röntgenuntersuchung einer Brust (O) ist in diesem Falle Teil der Steuereinrichtung 9 und umfasst die folgenden Komponenten (s. dazu auch das Verfahren gemäß Figur 2):

Eine Datenschnittstelle 11 ausgelegt zum Empfang von Bildern L, H der Röntgenuntersuchung, die nach Verabreichung eines Kontrastmittels aufgenommen worden sind, die Bilder umfassend zumindest ein LE-Bild L, welches mit einer vorgegebenen niedrigen Röntgenenergie aufgenommen worden ist ein HE-Bild H, welches mit einer vorgegebenen hohen Röntgenenergie aufgenommen worden ist.

Eine Jodbild-Einheit 12 ausgelegt zum Erstellen eines Jodbildes J aus dem LE-Bild L und dem HE-Bild H, z.B. mittels der Formel $\text{Pixel}_{Jod} = \ln(\text{Pixel}_{HE}) - w \cdot \ln(\text{Pixel}_{LE})$ mit dem Wichtigsfaktor w und dem natürlichen Logarithmus ln() für alle Pixel des Jodbildes.

Eine FGT-Einheit 13 ausgelegt zum Erstellen einer FGT-Maske F, in dem eine Klassifizierung für jedes Bildelement im LE-Bild L oder einer gewichteten Linearkombination des LE-Bildes und einer Anzahl weiterer spektraler Bilder durchgeführt wird, ob das Bildelement fibroglanduläres Gewebe darstellt, wobei im positiven Fall eine entsprechende Bildposition in der FGT-Maske F mit einem FGT-Marker M markiert wird.

Eine FGTE-Einheit 14 ausgelegt zum Erstellen einer FGT-E-Maske FE, in dem an den Bildpositionen im Jodbild J, deren Entsprechungen in der FGT-Karte F mit einem FGT-Marker M markiert sind, eine Klassifizierung stattfindet, ob dort eine Jod-Anreicherung vorliegt, wobei im positiven Fall eine entsprechende Bildposition in der FGT-E-Maske FE mit einem FGTE-Marker ME versehen wird.

Eine BPE-Einheit 15 ausgelegt zum Berechnen des volumetrischen BPE als Summe aller Bildpunkt-Werte im Jodbild J bei denen an der entsprechenden Stelle der FGT-E-Maske FE ein FGTE-Marker ME vorliegt, und ausgelegt zum Ermitteln von BPE-Ergebnisdaten BE basierend auf dem Jodbild J und dem volumetrischen BPE.

In diesem Beispiel ist die Datenschnittstelle 11 auch zum Ausgeben der BPE-Ergebnisdaten BE ausgelegt.

Figur 2 zeigt ein Blockdiagramm, welches beispielhaft den Ablauf eines erfindungsgemäßen Verfahrens zur Bestimmung des BPE in einer kontrast-verstärkten Röntgenuntersuchung einer Brust darstellt.

In Schritt I erfolgt eine Bereitstellung von Bildern L, H der Röntgenuntersuchung, die nach Verabreichung eines Kontrastmittels aufgenommen worden sind, die Bilder umfassend zumindest ein LE-Bild L, welches mit einer vorgegebenen niedrigen Röntgenenergie aufgenommen worden ist und ein HE-Bild H, welches mit einer vorgegebenen hohen Röntgenenergie aufgenommen worden ist.

In Schritt II wird ein Jodbild J aus dem LE-Bild L und dem HE-Bild H erstellt, z.B. mittels der Formel $\text{Pixel}_{Jod} = \ln(\text{Pixel}_{HE}) - w \cdot \ln(\text{Pixel}_{LE})$ mit dem Wichtigsfaktor w und dem natürlichen Logarithmus ln() für alle Pixel des Jodbildes.

In Schritt III wird eine FGT-Maske F erstellt, in dem eine Klassifizierung für jedes Bildelement im LE-Bild L durchgeführt wird, ob das Bildelement fibroglanduläres Gewebe darstellt, wobei im positiven Fall eine entsprechende Bildposition in der FGT-Maske F mit einem FGT-Marker M markiert wird.

In Schritt IV wird eine FGT-E-Maske FE erstellt, in dem an den Bildpositionen im Jodbild J, deren Entsprechungen in der FGT-Karte F mit einem FGT-Marker M markiert sind, eine Klassifizierung stattfindet, ob dort eine Jod-Anreicherung vorliegt, wobei im positiven Fall eine entsprechende Bildposition in der FGT-E-Maske FE mit einem FGTE-Marker ME versehen wird.

In Schritt V wird das volumetrische BPE berechnet. Das vBPE ist dabei die Summe aller Bildpunkt-Werte im Jodbild J bei denen an der entsprechenden Stelle der FGT-E-Maske FE ein FGTE-Marker ME vorliegt, normiert auf ein Volumen.

In Schritt VI werden BPE-Ergebnisdaten BE als eine Kategorisierung des volumetrischen BPE und eine bildliche Darstellung basierend auf der Jodkarte J und der FGT-E-Maske FE ermittelt und diese BPE-Ergebnisdaten BE ausgegeben.

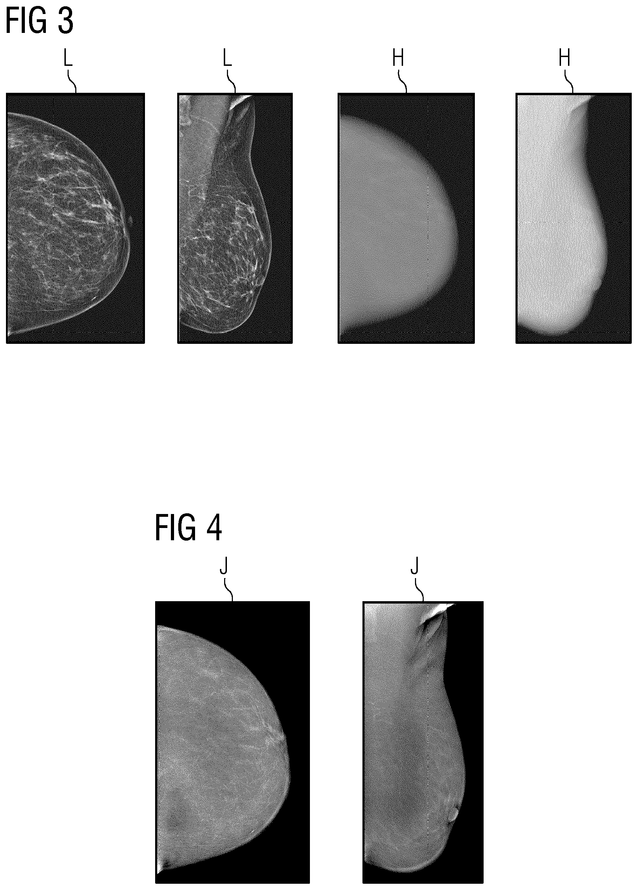

Figur 3 zeigt ein Beispiel für aufgenommene LE-Bilder L und HE-Bilder H einer Brust von oben und von der Seite. Diese Daten dienen als Grundlage für ein erfindungsgemäßes Verfahren.

Das erste und dritte Bild zeigen die Brust von oben, das zweite und vierte zeigen die Brust von der Seite.

Figur 4 zeigt ein Beispiel für Jodbilder J einer Brust basierend auf den LE-Bildern L und HE-Bildern H der Figur 3. Sie wurden mit der Funktion Jodbild = ln(HE-Bild)-w·ln(LE-Bild) erstellt, die oben genauer erklärt wird. Links sieht man das Jodbild J der Brust, die von oben aufgenommen wurde, rechts eine Seitenansicht.

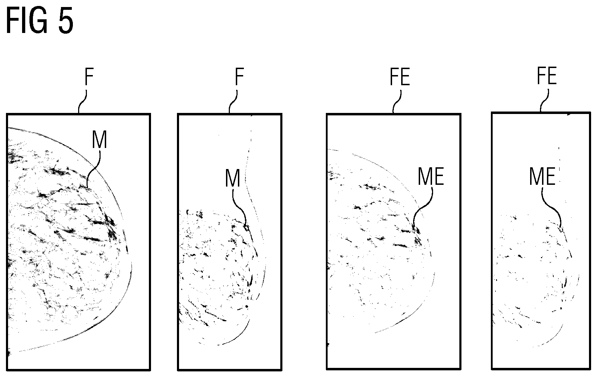

Figur 5 zeigt ein Beispiel für FGT-Masken F und FGT-E-Masken FE. Das erste und dritte Bild zeigen die Brust von oben, das zweite und vierte zeigen die Brust von der Seite. Schwarze Punkte repräsentieren Marker M, ME. Die FGT-Marker M in den FGT-Masken F sind ausgedehnter als die FGTE-Marker ME in den FGT-E-Masken FE, da für letztere nur die Einträge der FGT-Maske F verwendet werden, bei denen in den Jodbildern die Bildwerte (die den Jodanteil repräsentieren) über einer vorgegebenen Schwelle liegen mussten.

Es wird abschließend noch einmal darauf hingewiesen, dass es sich bei den vorhergehend detailliert beschriebenen Verfahren sowie bei dem dargestellten System lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen Begriffe wie "Einheit" nicht aus, dass die betreffenden Komponenten aus mehreren zusammenwirkenden TeilKomponenten bestehen, die gegebenenfalls auch räumlich verteilt sein können. Der Begriff "eine Anzahl" ist als "mindestens ein(e)" zu lesen.

**Patentansprüche**

**1.** Verfahren zur Bestimmung des "Background Parenchymal Enhancement", BPE, in einer kontrastmittelverstärkten Röntgenuntersuchung einer Brust umfassend die Schritte:

- Bereitstellen von Bildern (L, H) der Röntgenuntersuchung, die nach Verabreichung eines Kontrastmittels aufgenommen worden sind, die Bilder umfassend zumindest ein LE-Bild (L), welches mit einer vorgegebenen niedrigen Röntgenenergie aufgenommen worden ist und ein HE-Bild (H), welches mit einer vorgegebenen hohen Röntgenenergie aufgenommen worden ist,
- Erstellen eines Jodbildes (J) aus dem LE-Bild (L) und dem HE-Bild (H),
- Berechnen eines volumetrischen BPE als Summe aller Bildpunkt-Werte im Jodbild (J), normiert auf ein Volumen, für die die folgenden Bedingungen zutreffen:

i) es befindet sich dort im Jodbild (J) eine Jod-Anreicherung,
ii) es befindet sich dort fibroglanduläres Gewebe,

- Ermitteln von BPE-Ergebnisdaten (BE) basierend auf dem Jodbild (J) und dem volumetrischen BPE,
- Ausgeben der BPE-Ergebnisdaten (BE).

**2.** Verfahren nach Anspruch 1, wobei die Berechnung des volumetrischen BPE mittels der folgenden Schritte erfolgt:

- Erstellen einer FGT-Maske (F), in dem eine Klassifizierung für jedes Bildelement im LE-Bild (L) oder einer gewichteten Linearkombination des LE Bildes und einer Anzahl weiterer spektraler Bilder durchgeführt wird, ob das Bildelement fibroglanduläres Gewebe darstellt, wobei im positiven Fall eine entsprechende Bildposition in der FGT-Maske (F) mit einem FGT-Marker (M) markiert wird,
- Erstellen einer FGT-E-Maske (FE), in dem an den Bildpositionen im Jodbild (J), deren Entsprechungen in der FGT-Karte (F) mit einem FGT-Marker (M) markiert sind, eine Klassifizierung stattfindet, ob dort eine Jod-Anreicherung vorliegt, wobei im positiven Fall eine entsprechende Bildposition in der FGT-E-Maske (FE) mit einem FGTE-Marker (ME) versehen wird,
- Berechnen des volumetrischen BPE als Summe aller Bildpunkt-Werte im Jodbild (J) bei denen an der entsprechenden Stelle der FGT-E-Maske (FE) ein FGTE-Marker (ME) vorliegt.

**3.** Verfahren nach Anspruch 2, umfassend die zusätzlichen Schritte:

- Zählen der Anzahl von FGT-Markern (M) der FGT-Maske (F) als X und der Anzahl von FGTE-Markern (FE) der FGT-E-Maske (ME) als XE,

- Berechnen des relativen BPE rBPE mit dem Quotienten rBPE = XE / X.

4.  Verfahren nach einem der vorangehenden Ansprüche, wobei zusätzlich eine automatisierte Klassifikation von BPE-Ergebnisdaten im Jodbild (J) durchgeführt wird, bevorzugt wobei eine ortsabhängige Klassifikation durch Anwendung einer Zuordnungs-Funktion auf Bildelementen des Jodbilds (J) ermittelt wird, insbesondere einer Zuordnungs-Funktion, die Intervallen eines kontinuierlichen Wertebereichs konkrete Bezeichnungen zuweist, insbesondere vier Bezeichnungen, die vier unterschiedliche Stärkegrade angeben, oder einen anderen Wertebereich.

5.  Verfahren nach einem der vorangehenden Ansprüche, wobei im Rahmen der Ausgabe der BPE-Ergebnisdaten (BE) eine grafische Anzeige des Jodbildes (J) oder eines aus dem Jodbild (J) abgeleiteten Bildes erfolgt und/oder die Anzeige des volumetrischen BPE und/oder eines Wertes rBPE erfolgt und bevorzugt eine Kombination von Ergebnissen per Ansicht und per Brust.

6.  Verfahren nach einem der vorangehenden Ansprüche, wobei eine automatisierte Erkennung der Abweichung zwischen einer lokalen mammographischen Brustdichte und den BPE-Ergebnisdaten (BE) basierend auf dem LE-Bild (L) und dem Jodbild (J) erfolgt, wobei bei Nichtübereinstimmung des Musters des BPE und der Brustdichte ein Indikator für einen relevanten klinischen Zustand generiert wird,
    bevorzugt mit den Schritten:

    - Berechnen einer Brustdichtekarte aus dem LE-Bild (L) oder einer Linearkombination von LE-Bild (L) und HE-Bild (H),
    - Vergleichen der Intensitätswerte der Bildelemente der Brustdichtekarte mit den entsprechenden Bildelementen im Jodbild (J), wobei der verglichene Bildbereich bevorzugt durch die FGT-Maske (F) und/oder die FGT-E-Maske (FE) vorgegeben wird, unter Bildung einer Anzahl von Abweichungswerten, wobei in vorgegebenen Sub-Bereichen mittlere Abweichungswerte berechnet werden,
    - Ermitteln, ob die Abweichungswerte lokal oder global außerhalb eines vorgegebenen Wertebereichs liegen, insbesondere durch Anwendung einer Korrelationsanalyse.

7.  Verfahren nach einem der vorangehenden Ansprüche, wobei eine automatisierte Analyse der räumlichen Verteilung, Morphologie und Textur von BPE im Jodbild (J) erfolgt,

    - bevorzugt wobei ermittelt wird, ob die BPE lokalisiert oder homogen im Jodbild (J) verteilt ist, insbesondere wobei morphologische Operatoren sequentiell auf das Jodbild (J) oder die auf die FGT-E-Maske (FE) angewendet werden, und/oder
    - bevorzugt wobei die Morphologie und Textur der BPE ermittelt wird, insbesondere unter Verwendung von Haralick-Texturfunktionen, welche auf die FGT-E-Maske (FE) und/oder das Jodbild (J) angewendet werden,

    insbesondere wobei quantitative bildgebende Biomarker aus dem Jodbild (J), der FGT-E-Maske (FE), dem LE-Bild (L), dem HE-Bild (H) und/oder davon abgeleiteten Bildern oder Masken extrahiert werden.

8.  Verfahren nach einem der vorangehenden Ansprüche, wobei eine automatisierte Längsschnittbewertung von BPE basierend auf dem Jodbild (J) durchgeführt wird,
    bevorzugt wobei das Verfahren mit LE-Bildern (L) und HE-Bildern (H) mehrerer Röntgenuntersuchungen der gleichen Person durchgeführt wird und die relative Veränderung des volumetrischen BPE über die unterschiedlichen Röntgenuntersuchungen hinweg berechnet wird und oder eine Änderung der räumlichen Verteilung, der Morphologie und/oder der Textur von BPE berechnet wird.

9.  Verfahren nach einem der vorangehenden Ansprüche, wobei basierend auf LE-Bildern (L) und HE-Bildern (H) einer bilateralen mammographischen Untersuchung eine automatisierte Bewertung der Symmetrie basierend auf dem Jodbild (J) einer aufgenommenen rechten Brust und einer aufgenommenen linken Brust durchgeführt wird.

10. Verfahren nach einem der vorangehenden Ansprüche, wobei die Röntgenuntersuchung mittels eines spektralen Mehrenergie-Bildgebungsverfahrens erfolgte und das Jodbild (J) und/oder die FGT-E-Maske (FE) und/oder insbesondere die FGT-Maske (F) mittels mindestens eines weiteren Bildes generiert wird, welches bei einer anderen Energie als das LE-Bild (L) und das HE-Bild (H) aufgenommen wurde, und/oder wobei die BPE-Ergebnisdaten (BE) mittels kombinierten Bildern ermittelt werden, und/oder wobei die Röntgenuntersuchung eine tomographische Untersuchung ist, insbesondere eine kontrastmittelverstärkte Brust-CT.

11. System (10) zur Bestimmung des "Background Parenchymal Enhancement", BPE, in einer kontrastmittelverstärkten Röntgenuntersuchung einer Brust (O) gemäß einem Verfahren nach einem der vorangehenden Ansprüche, das System (10) umfassend:

- eine Datenschnittstelle (11) ausgelegt zum Empfang von Bildern (L, H) der Röntgenuntersuchung, die nach Verabreichung eines Kontrastmittels aufgenommen worden sind, die Bilder umfassend zumindest ein LE-Bild (L), welches mit einer vorgegebenen niedrigen Röntgenenergie aufgenommen worden ist und ein HE-Bild (H), welches mit einer vorgegebenen hohen Röntgenenergie aufgenommen worden ist,
- eine Jodbild-Einheit (12) ausgelegt zum Erstellen eines Jodbildes (J) aus dem LE-Bild (L) und dem HE-Bild (H),
- optional: eine FGT-Einheit (13) ausgelegt zum Erstellen einer FGT-Maske (F), in dem eine Klassifizierung für jedes Bildelement im LE-Bild (L) durchgeführt wird, ob das Bildelement fibroglanduläres Gewebe darstellt, wobei im positiven Fall eine entsprechende Bildposition in der FGT-Maske (F) mit einem FGT-Marker (M) markiert wird,
- optional: eine FGTE-Einheit (14) ausgelegt zum Erstellen einer FGT-E-Maske (FE), in dem an den Bildpositionen im Jodbild (J), deren Entsprechungen in der FGT-Karte (F) mit einem FGT-Marker (M) markiert sind, eine Klassifizierung stattfindet, ob dort eine Jod-Anreicherung vorliegt, wobei im positiven Fall eine entsprechende Bildposition in der FGT-E-Maske (FE) mit einem FGTE-Marker (ME) versehen wird,
- eine BPE-Einheit (15) ausgelegt zum Berechnen eines volumetrischen BPE als Summe aller Bildpunkt-Werte im Jodbild (J) für die die folgenden Bedingungen zutreffen:

i) es befindet sich dort im Jodbild eine Jod-Anreicherung,
ii) es befindet sich dort fibroglanduläres Gewebe, insbesondere ausgelegt zum Berechnen des volumetrischen BPE als Summe aller Bildpunkt-Werte im Jodbild (J), normiert auf ein Volumen, bei denen an der entsprechenden Stelle der FGT-E-Maske (FE) ein FGTE-Marker (ME) vorliegt,

und ausgelegt zum Ermitteln von BPE-Ergebnisdaten (BE) basierend auf dem Jodbild (J) und dem volumetrischen BPE,
- eine Datenschnittstelle (11) ausgelegt zum Ausgeben der BPE-Ergebnisdaten (BE).

12. Steuereinrichtung (9) ausgelegt zur Steuerung eines Mammographiesystems (1) umfassend ein System (10) nach Anspruch 11.

13. Mammographiesystem (1) umfassend eine Steuereinrichtung nach Anspruch 12.

14. Computerprogrammprodukt, umfassend Befehle, die bei der Ausführung des Programms durch einen Computer diesen veranlassen, die Schritte des Verfahrens nach Anspruch 1 bis 10 auszuführen.

15. Computerlesbares Speichermedium, umfassend Befehle, die bei der Ausführung durch einen Computer diesen veranlassen, die Schritte des Verfahrens nach Anspruch 1 bis 10 auszuführen.

**Claims**

1. Method for determining the "Background Parenchymal Enhancement", BPE, in a contrast medium-enhanced X-ray examination of a breast comprising the steps:

- providing images (L, H) of the X-ray examination, said images having been taken after administration of a contrast medium, the images comprising at least one LE image (L) which has been taken at a predetermined low X-ray energy and an HE image (H) which has been taken at a predetermined high X-ray energy,
- creating an iodine image (J) from the LE image (L) and the HE image (H),
- calculating a volumetric BPE as the sum of all pixel values in the iodine image (J), normalised to a volume, for which the following conditions apply:

i) iodine enrichment is present in the iodine image (J),
ii) fibroglandular tissue is present,

- obtaining BPE result data (BE) based on the iodine image (J) and volumetric BPE,
- outputting the BPE result data (BE).

**2.** Method according to claim 1, wherein the calculation of the volumetric BPE is performed by means of the following steps:

- creating an FGT mask (F) in which a classification is performed for each picture element in the LE image (L) or a weighted linear combination of the LE image and a number of further spectral images as to whether the picture element represents fibroglandular tissue, wherein in the positive case a corresponding image position in the FGT mask (F) is marked with an FGT marker (M),
- creating an FGT-E mask (FE), in which a classification is performed at the image positions in the iodine image (J) whose correspondences in the FGT map (F) are marked with an FGT marker (M) as to whether iodine enrichment is present there, wherein in the positive case a corresponding image position in the FGT-E mask (FE) is provided with an FGTE marker (ME),
- calculating the volumetric BPE as the sum of all pixel values in the iodine image (J) for which an FGTE marker (ME) is present at the corresponding position of the FGT-E mask (FE).

**3.** Method according to claim 2, comprising the additional steps:

- counting the number of FGT markers (M) of the FGT mask (F) as X and the number of FGTE markers (FE) of the FGT-E mask (ME) as XE,
- calculating the relative BPE rBPE with the quotient rBPE = XE / X.

**4.** Method according to one of the preceding claims, wherein in addition an automated classification of BPE result data in the iodine image (J) is performed, preferably wherein a location-dependent classification is determined by applying an assignment function to image elements of the iodine image (J), in particular an assignment function which assigns specific designations to intervals of a continuous range of values, in particular four designations which indicate four different degrees of strength, or another range of values.

**5.** Method according to one of the preceding claims, wherein in the context of the output of the BPE result data (BE) a graphical display of the iodine image (J) or of an image derived from the iodine image (J) is performed and/or the display of the volumetric BPE and/or of a value rBPE is performed and preferably a combination of results per view and per breast.

**6.** Method according to one of the preceding claims, wherein automated detection of the deviation between a local mammographic breast density and the BPE result data (BE) based on the LE image (L) and the iodine image (J) is performed, wherein if the pattern of the BPE and the breast density do not match, an indicator of a relevant clinical condition is generated,
preferably with the steps:

- calculating a breast density map from the LE image (L) or a linear combination of LE image (L) and HE image (H),
- comparing the intensity values of the image elements of the breast density map with the corresponding image elements in the iodine image (J), wherein the compared image area is preferably predetermined by the FGT mask (F) and/or the FGT-E mask (FE), with formation of a number of deviation values, wherein mean deviation values are calculated in predetermined sub-areas,
- determining whether the deviation values lie locally or globally outside a predefined range of values, in particular by applying a correlation analysis.

**7.** Method according to one of the preceding claims, wherein an automated analysis of the spatial distribution, morphology and texture of BPE in the iodine image (J) is performed.

- preferably wherein it is determined whether the BPE is localised or homogeneously distributed in the iodine image (J), in particular wherein morphological operators are applied sequentially to the iodine image (J) or to the FGT-E mask (FE).
- preferably wherein the morphology and texture of the BPE is determined, in particular using Haralick texture functions which are applied to the FGT-E mask (FE) and/or the iodine image (J),

in particular wherein quantitative imaging biomarkers are extracted from the iodine image (J), the FGT-E mask (FE), the LE image (L), the HE image (H) and/or images or masks derived therefrom.

**8.** Method according to one of the preceding claims, wherein an automated longitudinal section assessment of BPE is performed based on the iodine image (J),
preferably wherein the method is performed using LE images (L) and HE images (H) from multiple X-ray examinations of the same individual and the relative change in volumetric BPE across the different X-ray examinations is calculated and or a change in spatial distribution, morphology and/or texture of BPE is calculated.

9. Method according to one of the preceding claims, wherein based on LE images (L) and HE images (H) of a bilateral mammographic examination, an automated assessment of symmetry is performed based on the iodine image (J) of an imaged right breast and an imaged left breast.

10. Method according to one of the preceding claims, wherein the X-ray examination was performed by means of a spectral multi-energy imaging method and the iodine image (J) and/or the FGT-E mask (FE) and/or in particular the FGT mask (F) is generated by means of at least one further image which was taken at a different energy than the LE image (L) and the HE image (H), and/or wherein the BPE result data (BE) is determined by means of combined images, and/or wherein the X-ray examination is a tomographic examination, in particular a contrast medium-enhanced breast CT.

11. System (10) for determining the "Background Parenchymal Enhancement", BPE, in a contrast medium-enhanced X-ray examination of a breast (O) in accordance with a method according to one of the preceding claims, the system (10) comprising:

- a data interface (11) designed to receive images (L, H) of the X-ray examination, said images having been taken after administration of a contrast medium, the images comprising at least one LE image (L) which has been taken at a predetermined low X-ray energy and an HE image (H) which has been taken at a predetermined high X-ray energy,
- an iodine image unit (12) designed to create an iodine image (J) from the LE image (L) and the HE image (H),
- optional: an FGT unit (13) designed to create an FGT mask (F) in which a classification is performed for each picture element in the LE image (L) as to whether the picture element represents fibroglandular tissue, wherein in the positive case a corresponding image position in the FGT mask (F) is marked with an FGT marker (M),
- optional: an FGTE unit (14) designed to create an FGT-E mask (FE) in which a classification is performed at the image positions in the iodine image (J), whose correspondences in the FGT map (F) are marked with an FGT marker (M), as to whether an iodine enrichment is present there, wherein in the positive case a corresponding image position in the FGT-E mask (FE) is provided with an FGTE marker (ME),
- a BPE unit (15) designed to calculate a volumetric BPE as the sum of all pixel values in the iodine image (J) for which the following conditions apply:

i) iodine enrichment is present in the iodine image,
ii) fibroglandular tissue is present, in particular designed to calculate the volumetric BPE as the sum of all pixel values in the iodine image (J), normalised to a volume, for which an FGTE marker (ME) is present at the corresponding position of the FGT-E mask (FE),

and designed to obtain BPE result data (BE) based on the iodine image (J) and volumetric BPE,
- a data interface (11) designed to output the BPE result data (BE).

12. Control facility (9) designed to control a mammography system (1) comprising a system (10) according to claim 11.

13. Mammography system (1) comprising a control facility according to claim 12.

14. Computer program product comprising commands that, when the program is executed by a computer, cause said computer program product to perform the steps of the method according to claim 1 to 10.

15. Computer-readable storage medium comprising commands that, when executed by a computer, cause said computer to perform the steps of the method according to claim 1 to 10.


**Revendications**

1. Procédé de détermination du "background parenchymal enhancement", BPE, dans un examen aux rayons X, amélioré par un agent de contraste, d'un sein, comprend les stades :

- se procurer des images (L, H) de l'examen aux rayons X, qui ont été enregistrées après l'administration d'un agent de contraste, les images comprenant au moins une image LE (L), qui a été enregistrée à une énergie de rayons X petite donnée à l'avance et une image HE (H), qui a été enregistrée à une énergie de rayons X grande donnée à l'avance,
- établissement d'une image (J) iodée à partir de l'image LE (L) et de l'image HE (H),
- calcul d'un BPE volumétrique comme somme de toutes les valeurs de points d'image dans l'image (J) iodée, normée sur un volume, pour laquelle les conditions suivantes sont satisfaites :

> i) il s'y trouve dans l'image (J) iodée un enrichissement en iode,
> ii) il s'y trouve du tissu fibroglandulaire,

- déterminer des données (BE) de résultat BPE sur la base de l'image (J) iodée et du BPE volumétrique,
- donner les données (BE) de résultat BPE.

2. Procédé suivant la revendication 1, dans lequel le calcul du BPE volumétrique s'effectue au moyen des stades suivants :

- établissement d'un masque FGT (F), dans lequel on effectue un classement pour chaque élément d'image dans l'image LE (L) ou une combinaison linéaire pondérée de l'image LE et d'un nombre d'autres images spectrales, si l'élément d'image représente du tissu fibroglandulaire, dans lequel dans l'affirmative on repère une position d'image correspondante dans le masque FGT (F) par un repère FGT (M),
- établissement d'un masque FGT-E (FE), dans lequel aux positions d'images dans l'image (J) iodée, dont les correspondances dans la carte FGT (F) sont repérées par un repère FGT (M), a lieu un classement, s'il s'y trouve un enregistrement en iode, dans lequel dans l'affirmative une position d'image correspondante dans le masque FGT-E (FE) est pourvue d'un repère FGTE (ME),
- calcul du BPE volumétrique comme somme de toutes les valeurs de points d'image dans l'image (J) iodée pour lesquelles il y a, aux emplacements correspondants du masque FGT-E (FE), un repère FGTE (ME).

3. Procédé suivant la revendication 2, comprenant les stades supplémentaires :

- décompte du nombre de repères FGT (M) du masque FGT (F) comme X et du nombre de repères FGTE (FE) du masque FGT-E (ME) comme XE,
- calcul du BBE relatif rBPE par le quotient rBPE = XE / X.

4. Procédé suivant l'une des revendications précédentes, dans lequel on effectue supplémentairement un classement automatisé de données de résultat BPE dans l'image (J) iodée, dans lequel de préférence on détermine un classement en fonction de l'emplacement par application d'une fonction d'affectation aux éléments de l'image (J) iodée, en particulier d'une fonction d'affectation, qui a des désignations concrètes d'intervalles d'une plage de valeurs continue, en particulier quatre désignations, qui indiquent quatre degrés d'intensité différents, ou une autre plage de valeurs.

5. Procédé suivant l'une des revendications précédentes, dans lequel dans le cadre de la sortie des données (BE) de résultat BPE, a lieu une indication graphique de l'image (J) iodée ou d'une image dérivée de l'image (J) iodée et/ou l'indication du BPE volumétrique et/ou d'une valeur rBPE et de préférence une combinaison de résultats par vue et par sein.

6. Procédé suivant l'une des revendications précédentes, dans lequel a lieu une identification automatisée de l'écart entre une densité du sein mammographique locale et les données (BE) de résultat BPE sur la base de l'image LE (L) et de l'image (J) iodée, dans lequel, s'il n'y a pas coïncidence du modèle du BPE et de la densité du sein, on produit un indicateur d'un état clinique pertinent, comprenant de préférence les stades :

- calcul d'une carte de densité du sein à partir de l'image LE (L) ou d'une combinaison linéaire de l'image LE (L) et de l'image HE (H),
- comparaison des valeurs d'intensité des éléments d'image de la carte de densité du sein aux éléments d'image correspondants dans l'image (J) iodée, dans lequel on donne à l'avance la partie d'image comparée, de préférence par le masque FGT (F) et/ou le masque FGT-E (FE), avec formation d'un nombre de valeurs d'écart, dans lequel on calcule des valeurs d'écart moyennes dans des sous-plages données à l'avance,

- détermination du point de savoir, si les valeurs d'écart se trouvent localement ou globalement à l'extérieur d'une plage de valeurs donnée à l'avance, en particulier par application d'une analyse de corrélation.

7. Procédé suivant l'une des revendications précédentes, dans lequel une analyse automatisée de la répartition dans l'espace de la morphologie et de la texture de BPE dans l'image (J) iodée a lieu,

- dans lequel de préférence on détermine, si le BPE est localisé ou réparti de manière homogène dans l'image (J) iodée, dans lequel, en particulier on applique des opérateurs morphologiques séquentiellement à l'image (J) iodée ou au masque FGT-E (FE),
et/ou
- dans lequel de préférence on détermine la morphologie et la texture de BPE en utilisant, en particulier, des fonctions de textures Haralick, qui sont appliquées au masque FGT-E (FE) et/ou à l'image (J) iodée,

dans lequel, en particulier, on extrait un biorepère quantitatif donnant une image à partir de l'image (J) iodée du masque FGT-E (FE), de l'image LE (L), de l'image HE (H) et/ou de masques, qui s'en déduisent.

8. Procédé suivant l'une des revendications, dans lequel on effectue une évaluation automatisée en coupe longitudinale de BPE sur la base de l'image (J) iodée,
dans lequel de préférence on effectue le procédé avec des images LE (L) et des images HE (H) de plusieurs examens aux rayons X de la même personne et on calcule la variation relative du BPE volumétrique sur les examens aux rayons X différents et/ou on calcule une variation de la répartition dans l'espace de la morphologie et/ou de la texture de BPE.

9. Procédé suivant l'une des revendications précédentes, dans lequel sur la base des images LE (L) et des images HE (H) d'un examen mammographique bilatéral, on effectue une évaluation automatisée de la symétrie sur la base de l'image (J) iodée d'un sein droit enregistré et/ou d'un sein gauche enregistré.

10. Procédé suivant l'une des revendications précédentes, dans lequel l'examen aux rayons X a lieu au moyen d'un procédé d'imagerie à plusieurs énergies spectrales et on crée l'image (J) iodée et/ou le masque FGT-E (FE) et/ou, en particulier le masque FGT (F) au moyen d'au moins une autre image, qui a été enregistrée à une autre énergie que l'image LE (L) et que l'image HE (H), et/ou dans lequel on détermine les données (BE) de résultat BPE, au moyen d'images combinées, et/ou dans lequel l'examen aux rayons X est un examen de tomodensitométrie, en particulier une tomodensitométrie du sein améliorée par un agent de contraste.

11. Système (10) de détermination du "background parenchymal enhancement", BPE, dans un examen aux rayons X amélioré par un agent de contraste d'un sein (O) ou selon un procédé suivant l'une des revendications précédentes, le système (10) comprenant :

- une interface (11) de données conçue pour la réception d'images (L, H) de l'examen aux rayons X, qui ont été enregistrées après l'administration d'un agent de contraste, les images comprenant au moins une image LE (L), qui a été enregistrée à une petite énergie de rayons X donnée à l'avance et une image HE (H), qui a été enregistrée à une grande énergie de rayons X donnée à l'avance,
- une unité (12) d'image iodée conçue pour établir une image (J) iodée à partir de l'image LE (L) et de l'image HE (H),
- éventuellement : une unité FGT (13) conçue pour établir un masque FGT (F), dans lequel on effectue un classement pour chaque élément d'image dans l'image LE (L), si l'élément d'image représente du tissu fibroglandulaire, dans lequel dans l'affirmative, on repère une position d'image correspondante dans le masque FGT (F) par un repère FGT (M),
- éventuellement : une unité FGTE (14) conçue pour établir un masque FGT-E (E), dans lequel en les positions d'image dans l'image (J) iodée, dont des correspondances dans la carte FGT (F) sont repérées par un repère FGT (M), a lieu un classement s'il s'y trouve un enrichissement en iode, dans lequel dans l'affirmative on munit une position d'image correspondante dans le masque FGT-E (FE) d'un repère FGTE (ME),
- une unité BPE (15) conçue pour le calcul d'un BPE volumétrique comme somme de toutes les valeurs de points d'image dans l'image (J) iodée, pour laquelle des conditions suivantes sont satisfaites :

i) il s'y trouve dans l'image iodée, un enrichissement en iode,
ii) il s'y trouve du tissu fibroglandulaire conçue, en particulier, pour le calcul du BPE volumétrique comme somme de toutes les valeurs de points d'image dans l'image (J) iodée, normée sur un volume, dans lesquelles aux emplacements correspondants du masque FGT-E (FE), il y a un repère FGTE (ME),

et conçue pour la détermination de données (BE) de résultat BPE sur la base de l'image (J) iodée et du BPE volumétrique,
- une interface (11) de données conçue pour donner les données (BE) de résultat BPE.

12. Dispositif (9) de commande conçu pour la commande d'un système (1) de mammographie comprenant un système (10) suivant la revendication 11.

13. Système (1) de mammographie comprenant un dispositif de commande suivant la revendication 12.

14. Produit de programme d'ordinateur, comprenant des instructions, qui, lors de l'exécution du programme par ordinateur, font que celui-ci exécute les stades du procédé suivant la revendication 1 à 10.

15. Support de mémoire exploitable par ordinateur, comprenant des instructions, qui, lors de l'exécution par un ordinateur, font que celui-ci exécute le procédé suivant la revendication 1 à 10.

FIG 1

FIG 2

FIG 3

FIG 4

## FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2022003656 A1
- EP 3073924 A1

**In der Beschreibung aufgeführte Nicht-Patentliteratur**

- **SAVARIDAS et al.** Could parenchymal enhancement on contrast-enhanced spectral mammography (CESM) represent a new breast cancer risk factor? Correlation with known radiology risk factors. *Clinical Radiology*, 2017, vol. 72 (12)
- **SORIN et al.** Background Parenchymal Enhancement at Contrast-Enhanced Spectral Mammography (CESM) as a Breast Cancer Risk Factor. *Academic Radiology*, 2020, vol. 27 (9), 1234-1240
- **BERG et al.** Training Radiologists to Interpret Contrast-enhanced Mammography: Toward a Standardized Lexicon. *Journal of Breast Imaging*, 2021, vol. 3 (2)
- **HA et al.** Fully automated convolutional neural network method for quantification of breast mri fibroglandular tissue and background parenchymal enhancement. *J Digit Imaging*, 2019, vol. 32 (141)
- **SAHA et al.** Machine learning-based prediction of future breast cancer using algorithmically measured background parenchymal enhancement on high-risk screening MRI. *Journal of Magnetic Resonance Imaging*, 2019, vol. 50 (2), 456-464
- **BORKOWSKI et al.** Fully automatic classification of breast MRI background parenchymal enhancement using a transfer learning approach. *Medicine (Baltimore)*, 2020, vol. 17 (99), e21243
- **WEI et al.** Fully automatic quantification of fibroglandular tissue and background parenchymal enhancement with accurate implementation for axial and sagittal breast MRI protocols. *Medical Physics*, 2021, vol. 48 (1), 238-252

- **NAM et al.** Fully Automatic Assessment of Background Parenchymal Enhancement on Breast MRI Using Machine-Learning Models. *Journal of Magnetic Resonance Imaging*, 2021, vol. 53 (3), 818-826
- **LAIDEVANT et al.** Compositional breast imaging using a dual-energy mammography protocol. *Medical Physics*, 2010, vol. 37 (1), 164-174
- **MICHIELSEN et al.** Iodine quantification in limited angle tomography. *Medical Physics.*, 2020, vol. 47 (10)
- **GENNARO et al.** Quantitative Breast Density in Contrast-Enhanced Mammography. *J. Clin. Med.*, 2021, vol. 10 (15), 3309
- **FIESELMANN et al.** Volumetric breast density measurement for personalized screening: accuracy, reproducibility, consistency, and agreement with visual assessment. *Journal of Medical Imaging*, 2019, vol. 6 (3), 031406
- **EKPO ; MCENTEE.** Measurement of breast density with digital breast tomosynthesis-a systematic review. *British Journal of Radiology*, 2014, vol. 87 (1043)
- **RELLA et al.** Association between background parenchymal enhancement and tumor response in patients with breast cancer receiving neoadjuvant chemotherapy. *Diagnostic Intervential Imaging*, 2020, vol. 101 (10), 649-655
- **WANG et al.** Computerized Detection of Breast Tissue Asymmetry Depicted on Bilateral Mammograms: A Preliminary Study of Breast Risk Stratification. *Academic Radiology*, 2010, vol. 17 (10), 1234-1241